**Europäisches Patentamt**

**European Patent Office**

**Office européen des brevets**

(19)

(11) Veröffentlichungsnummer: **0 124 779**
**A2**

(12) ## EUROPÄISCHE PATENTANMELDUNG

(21) Anmeldenummer: **84103819.3**

(22) Anmeldetag: **06.04.84**

(51) Int. Cl.³: **G 01 N 33/56**
**G 01 N 33/74, C 07 C 103/52**

(30) Priorität: **07.04.83 US 482384**

(43) Veröffentlichungstag der Anmeldung:
**14.11.84 Patentblatt 84/46**

(84) Benannte Vertragsstaaten:
**CH DE FR GB IT LI NL**

(71) Anmelder: **THE GEORGE WASHINGTON UNIVERSITY**
**2121 I Street N.W.**
**Washington, D.C.(US)**

(72) Erfinder: **Goldstein, Allan L.**
**2795 - 28th Street N.W.**
**Washington, D.C., 20008(US)**

(72) Erfinder: **McClure, John**
**747 Leicester Lane**
**Houston, Texas 77034(US)**

(72) Erfinder: **Low, Teresa L. K.**
**9105 Bramble Place**
**Annandale, Virginia 22003(US)**

(72) Erfinder: **Naylor, Paul H.**
**15702 Penn Manor Lane**
**Bowie, Maryland 20716(US)**

(74) Vertreter: **Lederer, Franz, Dr. et al,**
**Patentanwälte Dr. Lederer Franz Meyer-Roxlau Reiner F.**
**Lucile-Grahn-Strasse 22**
**D-8000 München 80(DE)**

(54) Radioimmunverfahren für Thymosin beta 4.

(57) Es wird ein Radiommunverfahren zur Bestimmung von Thymosin ß₄ beschrieben. Das Verfahren besteht darin, dass man die Probe mit einer bekannten menge von radioaktiv markiertem Tyr-C13-thymosin ß₄ und einem Antikörper gegen Thymosin ß₄ versetzt, den Antigen/Antikörper-Komplex vom ungebundenen radioaktiv markierten Thymosin ß₄ abtrennt, das Ausmass der Radioaktivität im Antigen/Antikörper-Komplex bestimmt und mit einer Standardkurve vergleicht.

EP 0 124 779 A2

The George Washington University, Washington D.C., U.S.A.

GW 4093/67

Radioimmunverfahren für Thymosin $\beta_4$

Thymosin $\beta_4$ ist ein hitzestabiles, saures Polypeptid, das aus 43 Aminosäureresten zusammengesetzt ist. Dieses Thymushormon wurde aus Kalbthymus isoliert und seine Aminosäuresequenz wurde bestimmt. Thymosin $\beta_4$ ist eines der vielen Polypeptide, die in der Thymosinfraktion 5 enthalten sind, welche in der Regulierung, Differenzierung und Funktion der Thymus-abhängigen Lymphozyten (T-Zellen) partizipiert. Die Isolierung, Charakterisierung und die Verwendung von Thymosin $\beta_4$ wird in grösserem Detail in U.S. Patent Nr. 4,297,276 beschrieben.

Ein Immunoassay für ein Polypeptidhormon des Thymus, welches als Thymopoietin oder Thymin bekannt ist, wird in der U.S. Patentschrift Nr. 4,055,633 beschrieben. Im besonderen beschreibt dieses Patent einen Radioimmunassay für Thymopoietin, bei dem ein Antikörper verwendet wird, der durch ein Immunogen erzeugt wird, welches gereinigtes Thymopoietin umfasst, das unter Verwendung von Glutaraldehyd kovalent an einen immunogenen Träger, wie Rindergammaglobulin gekuppelt wird. Das in diesem Assay verwendete markierte Antigen ist vorzugsweise [125]Jod-Thymopoietin.

Klt/20.3.84

- 2 -

Es muss beachtet werden, dass Thymopoietin absolut nicht analog zu Thymosin $\beta_4$ ist, und zwar in der Struktur, Aminosäurezusammensetzung und Sequenz, biologischer Aktivität, physikalischer Eigenschaften und immunologischer Eigenschaften.

Ein Radioimmunassay für eine teilweise gereinigte Thymosinfraktion, und zwar Thymosinfraktion 5, von der nunmehr bekannt ist, dass sie ein Gemisch von einer Vielzahl von Polypeptiden enthält, wird durch Schulof et al. in Fed. Proc. 32, 1962 (1973) beschrieben. Vgl. in diesem Zusammenhang auch Goldstein et al., Fed. Proc. 33, 2053 (1974).

Im U.S. Patent Nr. 4,264,571 wird ein Radioimmunverfahren für Thymosin $\alpha_1$ beschrieben. Dieses Verfahren verwendet einen Antikörper, der erzeugt wurde durch ein Immunogen, das aus Thymosin $\alpha_1$ besteht, das kovalent mittels Glutaraldehyd an Hemocyanin gebunden wurde. Als Label wird $^{125}$Jod-Thymosin $\alpha_1$ verwendet, das durch Behandeln mit Bolton-Hunter-Reagens hergestellt wurde. Das Immunverfahren verwendet die Doppel-Antikörper-Methode, um den erhaltenen Niederschlag des Immunkomplexes zu bewerkstelligen. Ziegen-Antikaninchengammaglobulin wird als zweiter Antikörper verwendet.

Im U.S. Patent Nr. 4,339,427 wird ein verbessertes Radioimmunverfahren für Thymosin $\alpha_1$ beschrieben, bei dem synthetisches Thymosin $\alpha_1$ zur Erzeugung des Antikörpers verwendet wird und bei dem ein Analoges von Thymosin $\alpha_1$, nämlich (Tyr$^1$)-Thymosin $\alpha_1$ als markiertes Peptid verwendet wird.

Die vorliegende Erfindung betrifft ein Radioimmunverfahren zur Messung von Thymosin $\beta_4$.

Das bei diesem Verfahren verwendete Immunogen zur Herstellung der Antikörper wird durch kovalentes Binden von

Thymosin $\beta_4$ an einen immunologischen Träger erhalten. Die Quelle für Thymosin $\beta_4$ ist für die vorliegende Erfindung nicht kritisch. Geeignetes Thymosin $\beta_4$ kann von Fraktion 5 von verschiedenen Säugern erhalten werden. So z.B. kann Thymosin $\beta_4$ verwendet werden, das aus Fraktion 5 von Menschen, Rind, Schaf oder Schwein erhalten wurde. Dies ist möglich, da die Aminosäuresequenz von Thymosin $\beta_4$ dieser verschiedener Säuger homolog ist.

Alternativ und vorzugsweise wird Thymosin $\beta_4$ verwendet, das nach herkömmlichen Peptidsynthesen hergestellt wurde. So z.B. kann Thymosin $\beta_4$ verwendet werden, das durch Fest- oder Flüssigphaseverfahren hergestellt wurde.

Der Begriff "immunogenes Trägermaterial" umfasst Materialien welche in der Lage sind, unabhängig in einem Wirstier eine immunogene Reaktion hervorzurufen, und welches entweder direkt oder über die Bildung einer Peptid- oder Esterbindung zwischen freien Carboxyl-, Amino- oder Hydroxylgruppen kovalent an Thymosin $\beta_4$ gekuppelt werden kann. Thymosin $\beta_4$ kann auch an entsprechende Gruppen des immunogenen Trägermaterials durch eine konventionelle bifunktionelle Bindegruppe gekuppelt werden.

Das kovalente Kuppeln von Thymosin $\beta_4$ zum immunogenen Trägermaterial kann nach herkömmlicher Art und Weise erfolgen. So kann z.B. für ein direktes Kuppeln ein Carbodiimid, vorzugsweise Dicyclohexylcarbodiimid oder 1-Aethyl--3-(3-dimethylaminopropyl)carbodiimid als Kupplungsmittel verwendet werden. In dieser Kupplungsreaktion ist es wünschenswert, ein leicht saures Reaktionsmedium zu verwenden, z.B. ein Medium mit einem pH im Bereich von ungefähr 3-6,5, ganz besonders bevorzugt im Bereich von ungefähr 4-6,5.

Ein geeignetes bifunktionelles Kupplungsmittel ist ein $C_{2-7}$-Dialkanal, wie Glutaraldehyd. Das Kuppeln kann in herkömmlicher Weise erfolgen, wie z.B. bei Avrameas, in Immunochemistry $\underline{6}$, 43 (1969) beschrieben.

Das erhaltene Immunogen kann ohne weitere Reinigung oder, obwohl nicht notwendig, nach Dialyse zur Entfernung von nichtreagiertem $ß_4$ und Kupplungsmittel, verwendet werden.

Geeignete immunogene Trägermaterialien, welche bei der Herstellung des Immunogens gemäss vorliegender Erfindung verwendet werden können, umfassen Proteine, natürliche oder synthetische polymere Verbindungen wie Polypeptide, z.B. Polylysin oder Copolymere von Aminosäuren, Polysaccharide und dergleichen. Besonders bevorzugte Trägermaterialien sind Proteine und Polypeptide, insbesondere Proteine.

Die Art des Proteins, welches als immunogenes Trägermaterial zur Herstellung des Immunogens gemäss vorliegender Erfindung verwendet wird, ist nicht kritisch. Beispiele geeignete Proteine umfassen Säugerserumproteine, wie z.B. menschliches Gammaglobulin, menschliches Serumalbumin, Rinderserumalbumin, methyliertes Rinderserumalbumin, Kaninchenserumalbumin, Rindergammaglobulin und Pferdegammaglobulin oder Nichtsäugerproteine wie Hemocyanin, besonders Keyhole Limpet Hemocyanin (KLH). Anderer geeignete Proteine sind dem Fachmann bestens bekannt.

Das Immunogen gemäss vorliegender Erfindung kann zur Bildung von Antikörpern verwendet werden, die spezifisch gegen Thymosin $ß_4$ gerichtet sind, indem Wirtstieren das Immunogen, vorzugsweise in Gegenwart eines Adjuvans injiziert wird. Erhöhte Titer können durch wiederholte Injektionen über eine bestimmte Zeitspanne erhalten werden. Geeignete Wirtstiere für diesen Zweck umfassen Säugetiere, wie Kaninchen, Pferde, Ziegen, Meerschweinchen, Ratten, Kühe, Schafe, etc. Die erhaltenen Antiseren enthalten Antikörper, welche selektiv mit Thymosin $ß_4$ komplexieren. Be-

dingt durch das hohe Ausmass der Homologie zwischen Thymosin $\beta_4$-Sequenzen hergeleitet von verschiedenen Säugern ist es möglich, Antikörper zu verwenden, die in einer Spezies von Thymosin $\beta_4$ erzeugt wurden, um Thymosin $\beta_4$ einer andern Säugespezies nachzuweisen.

Tyr-C13-Thymosin $\beta_4$ wird als Substrat für die Radioiodinierung verwendet. Das Ausdruck Tyr-C13-Thymosin $\beta_4$ bezieht sich auf ein Polypeptid enthaltend die 13 C-termi-nalen Aminosäuren von Thymosin $\beta_4$ mit Tyrosin gebunden an das N-terminale Ende. Die Verwendung dieses Analogen bedeutet einen echten Fortschritt gegenüber Methoden unter Verwendung des ganzen Moleküls, da diese entweder die Verwendung von Bolton-Hunter Radiomarkierungstechniken oder chemischen Modifikationen mit nicht-markiertem Bolton--Hunter-Reagens gefolgt durch klassische Radiomarkierungtechnik verlangen. Das Analogie liefert ein markiertes Produkt hoher spezifischer Aktivität, welches ein hohes Ausmass an Immunreaktivität behält. Der Thyrosinrest, der am terminalen Ende in der Sequenz eingeführt wird, bedingt eine kleinere sterische Hinderung beim Binden an den Antikörper als diejenige die erreicht würde, wenn man einen aromatischen Ring intern und möglicherweise nahe vor antigenischen Determinanten hinzufügen würde. Im weitern wurde gefunden, dass Tyr-C13-Thymosin $\beta_4$ einheitlicher und reproduzierbarer als natürliches Thymosin $\beta_4$ mit Bolton--Hunter-Reagens markiert werden kann. Die Verwendung chemisch-synthetisierter Peptide in diesem Verfahren ist bevorzugt. Dies gibt dem Verfahren einen hohen Grad von Spezifität, da keine Möglichkeit einer Kontamination des Präparates mit Verbindungen besteht, welche mit dem Ausgangsgewebe mitgereinigt werden könnten. Tyr-C13- Thymosin $\beta_4$, welches als Substrat für die Radiojodierung verwendet wird, kann unter Verwendung bekannter Festphasen Peptidsynthesen hergestellt werden, wie wenn man Thymosin $\beta_4$ herstellt, mit der Ausnahme, dass nur 13-Carboxy-terminale Aminosäuren von Thymosin $\beta_4$ hergestellt werden und dass als letzte Aminosäure Tyrosin zugefügt wird.

Obwohl radiojodiertes Tyr-C13-Thymosin $\beta_4$ mit Vorteil im Radioimmunverfahren verwendet wird, ist es möglich andere radioaktivmarkierte Reagenzien wie $(Tyr^1)$-Thymosin $\beta_4$ oder $(Tyr^1)$-Desacetylthymosin $\beta 4$ zu verwenden, welche mit $Jod^{125}$ oder Kohlenstoff 14 $(^{14}C)$ markiert werden. Durch bekannte Isotopenaustauschverfahren kann Tritium in diese Reagenzien eingefügt werden. Die Herstellung von $^{14}C$-(Tyr)C13-Thymosin $\beta_4$ oder $^{14}C$-$(Tyr^1)$-Desacetyl-thymosin $\beta_4$ oder $^{14}C$-$(Tyr^1)$-Thymosin $\beta_4$ kann einfach bewerkstelligt werden, indem man eine oder mehrere erhältliche $^{14}C$-markierte Aminosäuren an geeigneten Stellen der Festphasensynthese einführt.

Verschiedene Nachweismethoden können gemäss vorliegender Erfindung verwendet werden. In einer dieser Methoden werden bekannte Mengen einer zu untersuchenden Probe, Thymosin $\beta_4$ spezifischer Antikörper und markiertes Thymosin $\beta_4$ gemischt und stehengelassen. Der Antigen--Antikörperkomplex wird von den ungebunden Reagenzien durch bekannte Verfahren entfernt, z.B. durch Behandeln mit Ammoniumsulfat; Polyäthylenglykol; einem zweiten Antikörper, der entweder im Ueberschuss vorhanden ist, oder an eine unlösliche Phase gebunden ist; mit Dextran oder beschichteter Aktivkohle und dergleichen. Die Konzentration von markiertem Thymosin $\beta_4$ oder vom Thymosin $\beta_4$-Fragment wird entweder in der gebundenen oder ungebundenen Phase bestimmt, und der Thymosin $\beta_4$-Gehalt der Probe kann dann entweder in der gebundenen oder in der ungebundenen Phase bestimmt werden und zwar durch Vergleich der Menge an markierter Komponente verglichen mit einer in herkömmlicher Weise erhaltenen Standardkurve. Eine geeignete Standardkurve kann durch Mischen bekannter Mengen von Thymosin $\beta_4$ mit festen Mengen von markiertem Thymosin $\beta_4$ und Thymosin $\beta_4$-spezifischen Antikörper und Bestimmen des Ausmasses der Bindung für jede Menge erhalten werden.

Falls erwünscht, kann der Antikörper mit zahlreichen natürlichen Methoden zur Erhöhung der Spezifität behandelt werden. Ein geeignetes Behandlungsmittel ist Thymosin-- Fraktion 5 jedes Säugerorgans, welches keine Thymosin $\beta_4$-produzierende Zellen enthält. Geeignete Organe umfassen Nieren, Leber und Hirn. Säuger als Quellen für derartige Organe umfassen Kamele, Schafe, Pferde, Esel, Schweine, Menschen und dergleichen.

Die vorliegende Erfindung wird durch die nachfolgenden Beispiele weiter illustriert. In diesen Beispielen bedeutet "Boc" die Schutzgruppe t-Butyloxycarbonyl, "Bzl" die Schutzgruppe Benzyloxycarbonyl und "2-ClZ" die Schutzgruppe 2-Chlorbenzyloxycarbonyl.

## Beispiel 1

### Herstellung von Tyr-C13-Thymosin $\beta_4$:
### Tyr-Lys-Glu-Thr-Ile-Glu-Gln-Glu-Lys-Gln-Ala-Gly-Glu-Ser

Boc-Ser(Bzl)-OCH$_2$-C$_6$H$_4$-Harz (2,5 g; 1,0 mMol) wird in ein Peptidsynthesekessel gegeben, und die Festphasensynthese wird mit den nachfolgenden Schritten in jedem Zyklus durchgeführt: (1) drei Waschungen mit CH$_2$Cl$_2$. (2) Vorwaschen mit 40%iger Trifluoressigsäure (TFA) in Methylenchlorid. (3) Rühren während 28 Minuten mit 40%iger TFA in Methylenchlorid. (4) dreimaliges Waschen mit Methylenchlorid. (5) Vorwaschen mit 10%igem Triäthylamin (Et$_3$N) in Methylenchlorid. (6) Rühren während 8 Minuten mit 10%igem Et$_3$N in Methylenchlorid. (7) dreimaliges Waschen mit Methylenchlorid. (8) Rühren während 120 Minuten mit Boc-Glu(OBzl)-OH (3 mMol; 1,01 g) und Dicyclohexylcarbodiimid (DCC) (3 mMol; 0,62 g). (9) dreimaliges Waschen mit Methylenchlorid, 50%igem Isopropylalkohol in Methylenchlorid und dann Methylenchlorid.

Der Synthesezyklus wird unter Verwendung der folgenden Aminosäuren sequenziell wiederholt, in Schritt (8): Boc-Gly-OH, Boc-Ala-OH, Boc-Gln-OH, Boc-Lys(2-ClZ)-OH, Boc-Glu(OBzl)-OH, Boc-Gln-OH, Boc-Glu(OBzl)-OH, Boc-Ile-OH, Boc-Thr(Bzl)-OH, Boc-Glu(OBzl)-OH, Boc-Lys(2-ClZ)-OH, Boc-Tyr(Bzl), und 1-Hydroxybenzotriazol (HOBT, 6 mMol; 0,81 g) wird zu jeder Kupplungsreaktion gegeben, bei der Boc-Gln-OH in Schritt (8) involviert ist. Nach Beendigung der Synthese erhält man 4,14 g geschütztes Peptidharz. Dieses wird dann während 0°C während 30 Minuten einer HF-Spaltung (100 ml wasserfreies HF in 10 ml Anisol) unterworfen. Nach Entfernung von überschüssigem HF wird das Peptidharz mit 1%iger Essigsäure extrahiert und lyophilisiert und liefert 1,7 g rohes Produkt als leicht beiges gefärbtes Pulver. Das Produkt wird dann durch Hochdruckflüssigchromatographie (HPLC) an einer µBondapak C18-Kolonne (10-µm, 0,39 x 30 cm) bei 35°C gereinigt. Der Puffer in Reservoir A war 0,05% TFA (pH 2-3) und in Reservoir B Acetonitril enthaltend 0,05% TFA. Die Peptide wurden durch UV-Absorption bei 210 nm bestimmt. Die Durchflussgeschwindigkeit wurde auf 1,5 ml/Min. eingestellt. Die Peptide werden mit 5% B während 10 Minuten von der Kolonne eluiert gefolgt durch einen linearen Gradienten 45 bis 30% B in 50 Minuten. Tyr-C13-Thymosin $\beta_4$ wird bei 28 Minuten von der Kolonne eluiert. Aminosäureanalyse: Glu, 6,09; Thr. 0,85; Ser, 0,866; Gly, 1,08; Ala, 1,12; Ile, 0,90; Tyr, 0,83; Lys, 2,07.

### Beispiel 2

a) Herstellung des Antiserums. Synthetisches Thymosin $\beta_4$ (gesamtes Molekül von 43 Resten) wird mittels Glutaraldehyd kovalent an KLH gebunden. Synthetisches Thymosin $\beta_4$ (2,75 mg) und Keyhole Limpet Hemocyanin (KLH, 4,14g) werden mit 1,5 ml 0,25M Natriumphosphat vom pH 7,4 in ein geschlossenes 12 x 75 mm Plastikröhrchen gegeben. Durch die Zugabe von 75 µl 25%igem wässrigen Glutaraldehyd wird eine endgültige Konzentration von 1,25% Glutaraldehyd (W/V) erreicht. Nach leichtem Schütteln des Reaktionsgefässes während 3 Stunden bei Raumtemperatur wird die Lösung mit steriler physiologischer Kochsalzlösung (0,15M Natriumchlorid) zu einer Konzentration von 50 µg/ml Thymosin $\beta_4$ verdünnt. Das Gemisch der Reaktionsprodukte wird ohne weitere Reinigungen zur Immunisierung verwendet.

Weisse New Zealand-Kaninchen werden mit 50 µg synthetischem Thymosin $\beta_4$, welches an KLH konjugiert ist, an 20-30 intradermalen Stellen im Rücken nach der Methode von Vaitukaitis et al. (J. Clin. Endo, 33, 988 (1971) immunisiert. Damit jedes Kaninchen 50 µg Antigen in 2 ml Emulsion erhält, wird eine Emulsion hergestellt, die gleiche Mengen des wässrigen Proteins und Freund's vollständiges Adjuvans enthält. Während einer 4-Monats-Periode werden jede zwei Wochen Booster-Injektionen von 50 µg Thymosin

$\beta_4$ (als KLH-Konjugat) in jedes Tier injiziert (d.h. eine primäre Immunisierung gefolgt von 8 Booster-Injektionen). 14 Tage nach der achten Booster-Injektion wird erstmals Blut entnommen. Es werden monatlich weitere Booster-Injektionen gegeben, und es wird 10 Tage gewartet bevor die Blutentnahmen zur Bildung des Antiserums für den Immunassay vorgenommen werden.

b) <u>Radiojodierung des (Tyr)-C13-Thymosin $\beta_4$-Analogen.</u> Es wird eine Modifikation der Chloramin T-Methode zur Jodierung des (Tyr)-Thymosin $\beta_4$-Analogen verwendet (Greenwood, F.C., Hunter, W.M. and Glover, J.S., Biochem. J. <u>89</u>, 114 (1963). Das $\beta_4$-Analoge wird in 0,5M Phosphatpuffer (pH 6,0) bei einer Konzentration von 166 µg/ml) in Lösung gebracht. Zu 12 µl des Analogen werden 5 mCi NaI$^{125}$ in 20 µl Phosphatpuffer zugegeben. Chloramin T wird bei 0,5 mg/ml Phosphatpuffer hergestellt, und es werden 10 µl unter Mischen zugegeben. Nach 90 Sekunden werden 100 µl Metabisulfat (4 nMol/100 µl/ml; 7,6 µg/ml) zur Beendigung der Reaktion zugegeben. Um ein effizientes Ueberführen auf die G-10-Kolonne, welche für die Abtrennung von freiem $^{125}$I und markiertem Peptid nötig ist, zu bewerkstelligen, werden 50 µl normales Serum vor der Ueberführung zum Reaktionsröhrchen gegeben.

Die Sephadex G-10-Kolonne (0,7 x 10 cm) wird mit 10%iger Essigsäure mit 0,1% Eialbumin äquilibriert. Es werden 1 ml Aliquote gesammelt und das markierte Peptid wird als Tracer verwendet. Der Tracer wird in aliquote Anteile (5 µl) gegeben und sofort tiefgefroren. Vor dem Verfahren wird der Tracer auf 10'000 cpm/50 µl verdünnt. Nach 4 Wochen war eine Rechromatographie des Tracers möglich, was die Brauchbarkeit der einfachen Markierung auf 6-8 Wochen erweitert.

c) <u>Protokoll des Radioimmunverfahrens.</u> Es wird eine Stammlösung von Thymosin $\beta_4$ in einem Radioimmunverfahrenpuffer

(RIAB) in einer Konzentration hergestellt, welche im Arbeitsbereich des Verfahrens liegt, d.h. zwischen 0,5 und 37,5 ng/100 µl) und bei -20°C gefroren. Der RIAB war Phosphat-gepufferte Kochsalzlösung (pH 7,4, 0,01M Natriumphosphat und 0,15M Natriumchlorid) zu dem 0,05% (g/w) Natriumazid, 0,01 mM EDTA und Ammoniumsulfat fraktioniertes normales Kaninchenserum (NRS) zu einer Schlussverdünnung von 1/200 gegeben wurde. Das NRS dient sowohl als Protein zur Vermeidung nicht-spezifischer Bindung von Tracer und als Träger im Doppel-Antikörper-Fällungsschritt. Neun Standards enthaltend 0,5 bis 37,5 ng/100 µl werden aus der Stammlösung hergestellt und bei -70°C gefroren. Für unbekannte Proben wurden 5-20 µl Serum pro Verfahren verwendet, die mit Salzlösung zu 100 µl/Probe verdünnt wurden. Alle Röhrchen werden auf ein endgültiges Volumen von 400 µl mit RIAB eingestellt. Ein 50 µl Aliquot von Stammantiserum (1/200 Verdünnung) wird zu jedem Röhrchen gegeben. Die Verdünnung ergibt 20-25% Bindung von Tracer und eine endgültige Verdünnung von 1/2000. Nicht-spezifische Bindung wurde dadurch abgeschätzt, dass alle Röhrchen enthaltend alle Verfahrensreagenzien mit Ausnahme des spezifischen Anti-Thymosin $\beta_4$-Antiserums untersucht wur-den. Die Röhrchen werden am Vortex gemischt und während 24 Stunden bei 4°C inkubiert. Die Trennung von freiem und gebundenem Tracer wird durch die Zugabe von 50 µl einer Ziegenantikaninchen IgG-Präparation bewerkstelligt, die so eingestellt wurde, dass sie maximale Fällung des Tracers bewirkt. Nach Zugabe des zweiten Antikörpers und Mischen am Vortex werden die Proben über Nacht bei 4°C inkubiert. Das Immunpräzipitat wird bei 15000 x G während 25 Minuten bei 4°C zentrifugiert. Die Ueberstände werden abgesaugt und verworfen. Die Radioaktivität in den Immunpräzipitaten wird in einem automatischen Gammaspektrometer gemessen. Die Counts pro Minute für Standards und unbekannte Proben ($B_i$) werden bezüglich des nicht-spezifischen Hintergrundes, gewöhnlich 5% der gesamten Radioaktivität, korrigiert und durch die korrekte Zahl von Counts pro Minute dividiert, die bei Röhrchen erhalten wird, in denen kein
kompetitives Antigen zugefügt wird ($B_0$). Die Daten werden
mit einem Beckman-System DP-5500 analysiert, welcher die
Logdosis (x Achse) gegen die beobachteten Counts aufträgt
und die vier Parameter logistische Methode berechnet, bei
der die Dosis-Antwortskurve wie folgt angegeben wird:

$$Y = \frac{(A - D)}{1 + (x/C)B + D}$$

In dieser Gleichung bedeutet y die Antwort, x die Konzentration, A die Antwort wenn x = O(Bo), B ist der Steigungsfaktor, C die Dosis entsprechend der Antworthalbwertszeit
zwischen A und D, und D die Antwort für eine unbestimmte
Konzentration. Rodbard D. et al., Radioimmunoverfahren und
verwandte Verfahren in Med. 1, 469 (1978).

d) Resultate unter Verwendung des Radioimmunverfahrens für
Thymosin $\beta_4$. Synthetisches Thymosin $\beta_4$ kann unter Verwendung von Antikörper und $^{125}$J-Tyr-C13-Thymosin
$\beta_4$-Analog über ein Bereich von 0,4-37,5 ng/Röhrchen
(Figur 1) gemessen werden. Die Thymosin $\beta_4$-Spiegel in den
Seren von Menschen, Rindern, Mäusen, Hamstern und Meerschweinchen waren parallel zu den Standardkurven bei denen
zwischen 1 und 10 µl Thymosin verwendet wird. Der minimale auffindbare Serumspiegel war 5 mg/ml und eine Endkonzentration von 1/2000 Antikörper wurde verwendet, um eine
20-25%ige Bindung von Tracer am Antikörper zu erzielen.

Die initiale Spezifität des Radioimmunverfahrens wird
auf Kreuzreaktion überprüft durch Nachweisen von Präparaten
mit verschiedenen mutmasslichen Thymushormonen und verschiedenen Serumproteinen (Tabelle 1). In den Verfaren für
die Präparationen wurden ansteigende Mengen jedes Proteins
zum RIA-System zugegeben bis Spiegel erreicht wurden, die
eindeutig über den bekannten physiologischen Konzentrationen in Blut lagen oder bis ein praktischer Wert erreicht wurde, der durch die Menge von jeder erhältlichen Präparation bestimmt wurde. Für diejenigen Präparate die keine Antwort erzeugten, die vom Null-Dosisspiegel differierten, welche als 20%ige Inhibition bewertet wurden, wurde die grösste gemessene Dosis angegeben. Nur synthetisches Thymosin $\beta_4$ und Tyr-Cl3-Thymosin $\beta_4$-Analoges verdrängten den Tracer bei Spiegeln im 0,05 ng Bereich. Prealbumin zeigte bei Spiegeln von 100 µg/Röhrchen keine Kreuzreaktion. Thymopoietin und Thymosin $\alpha_7$ verdrängten 20% des Tracers bei Spiegeln von 10 µg. Thymosin $\alpha_1$, welches in Serum in pg-Mengen vorhanden ist, zeigte bei 100 ng/- Röhrchen keine Kreuzreaktion.

## Tabelle 1
### Spezifität des RIA für Thymosin $\beta_4$

| Getestetes Protein | benötigte Menge, um mindestens 20% $^{125}$Jod Tyr-Cl3-Thymosin $\beta_4$ zu verdrängen |
|---|---|
| A. Nicht-Thymusproteine und Peptide | |
| Hämocyanin (KLH) | > 100 µg |
| Albumin (menschlich) | > 100 µg |
| Hämoglobulin (menschlich) | > 100 µg |
| Myoglobulin (Pferd) | > 100 µg |
| Polyasparagin | > 100 µg |
| Prolactin | > 10 µg |
| Prealbumin | > 100 µg |
| B. Thymuspeptide | |
| Thymopoietin II | 10 µg |
| Thymosin $\alpha_7$ | 10 µg |
| Thymosin $\beta_4$ | 0,1 ng |
| Thymosin $\beta_4(C_{14})$ | 0,1 ng |
| Thymosin $\alpha_1$ | 1 µg |

Die Spiegel von in menschlichem Serum zirkulierendem Thymosin $\beta_4$ werden in Tabelle 2 gezeigt. Obwohl die Genauigkeit des Nachweisverfahrens innerhalb des erwarteten Bereiches liegt, schwanken die normalen Spiegel erheblich zwischen den einzelnen Individuen.

### Tabelle 2
Thymosin $\beta_4$ Spiegel in menschlichen Serum

| Quelle des Serums | Alter(Jahre) | Zahl | Thymosin (ng/ml) |
|---|---|---|---|
| männliches Blut | 25-50 | 33 | 850±249* |
| weibliches Blut | 25-50 | 23 | 700±168 |
| Nabelblut | Neugeborene | 20 | 1840±154 |

* ausgedrückt als Durchschnitt ± Standardabweichung

- 15 -

0124779

## Patentansprüche

1. Tyr-C13-Thymosin $\beta_4$.

2. Ein radioaktiver Tracer, welcher in einer Bestimmung für Thymosin $\beta_4$ verwendbar ist, der ein Polypeptid ausgewählt aus der Gruppe von Tyr-C13-Thymosin $\beta_4$, (Tyr)-Desacetyl-Thymosin $\beta_4$ und $(Tyr^1)$-Thymosin $\beta_4$ enthält, welches Polypeptid an ein Radioisotop gebunden ist.

3. Ein radioaktiver Tracer wie in Anspruch 2 beansprucht, dadurch gekennzeichnet, dass er $^{125}$Jod-(Tyr)--C13-Thymosin $\beta_4$ ist.

4. Immunogen enthaltend Thymosin $\beta_4$, welches kovalent an ein immunologisches Trägermaterial gebunden ist.

5. Ein Immunogen gemäss Anspruch 4, dadurch gekennzeichnet, dass der immunologische Träger Keyhole Limpet Hämocyanin ist.

6. Ein Radioimmunverfahren für Thymosin $\beta_4$ in einer Probe, welches die nachfolgenden Schritte umfasst:

(a) Inkubieren der Probe mit einer bekannten Menge von radioaktiv markiertem Tyr-C13-Thymosin $\beta_4$ und einem Antikörper, welcher selektiv mit Thymosin $\beta_4$ komplexiert,

(b) Trennung des erhaltenen Antikörper-Antigenkomplexes von ungebundenem radioaktiv markierten Thymosin $\beta_4$,

(c) Messen des Ausmasses der Bindung von radioaktiv markiertem Thymosin $\beta_4$ in diesem Komplex und

(d) Bestimmen der Menge von Thymosin $\beta_4$ in der Probe durch Vergleich des Grades der Bindung mit einer Standardkurve.

7. Radioimmunverfahren gemäss Anspruch 6, dadurch gekennzeichnet, dass das radioaktiv markierte Tyr-C13--Thymosin $\beta_4$ $^{125}$Jod-(Tyr)-C13-Thymosin $\beta_4$ ist.

8. Radioimmunverfahren gemäss Anspruch 7, dadurch gekennzeichnet, dass der Antikörper ein Antikörper ist, welcher in einem Säugetier als Antwort auf ein Immunogen enthaltend Thymosin $\beta_4$, welches kovalent an ein immunologisches Trägermaterial gebunden wird, gebildet wird.

9. Radioimmunverfahren gemäss Anspruch 8, dadurch gekennzeichnet, dass das immunologische Trägermaterial Keyhole Limpet Hämocyanin ist.

10. Ein Antikörper, welcher das Polypeptid-Thymosin $\beta_4$ selektiv erkennt und bindet.

11. Antikörper gemäss Anspruch 10, der in einem Wirtstier gebildet wird, dem Thymosin $\beta_4$, welches kovalent an ein immunogenes Material gebunden wird, injiziert wurde.

12. Antikörper gemäss Anspruch 11, dadurch gekennzeichnet, dass das immunogene Trägermaterial Keyhole Limpet Hämocyanin ist.

***